# EUROPEAN PATENT SPECIFICATION

(11) **EP 0 682 950 B1**
(45) Date of publication and mention of the grant of the patent: **21.07.1999**
(21) Application number: 95303365.1
(22) Date of filing: 19.05.1995
(51) Int. Cl.: A61K 39/09, A61K 9/00, C07K 14/315

(54) **Mucosal administration of pneumococcal antigens**
Mukosale Verabreichung von Pneumokokken-Antigenen
Administration mucosale d'antigènes de pneumococcus

(30) Priority: 20.05.1994 US 246636; 30.09.1994 US 312949
(43) Date of publication of application: 22.11.1995
(73) Proprietor: UAB RESEARCH FOUNDATION, Birmingham, AL 35294-009 (US)
(72) Inventor: Briles, David E., Birmingham, Alabama 35222 (US); Wu, Hong-Yin, Birmingham, Alabama 35216 (US)
(74) Representative: Smart, Peter John

(56) References cited:
- EP-A- 0 622 081
- WO-A-92/14488
- WO-A-94/14318
- INFECTION AND IMMUNITY, vol. 56, no. 10, 1988 pages 2666-2672, BESSEN,D. ET AL 'Influence of Intranasal Immunization with Synthetic Peptides Corresponding to Conserved Epitopes of M Protein on Mucosal Colonization by Group A Streptococci'
- MICROBIAL PATHOGENESIS, vol.1, no., 1986, page 519 - 531, L. S. MCDANIEL ET AL. 'ANALYSIS OF A SURFACE PROTEIN OF STREPTOCOCCUS PNEUMONIAE RECOGNISED BY PROTECTIVE MONOCLONAL ANTIBODIES.'

## Description

This invention relates to mucosal immunisation of animals with pneumococcal antigens to provide protection against pneumococcal colonisation and systemic infection.

Streptococcus pneumoniae causes more fatal infections world-wide than almost any other pathogen (refs. 1, 2, - a list of the references appears at the end of the disclosure). In the U.S.A., deaths caused by S. pneumoniae rival in numbers those caused by AIDS (ref. 1). In the U.S.A., most fatal pneumococcal infections occur in individuals over 65 years of age, in whom S. pneumoniae is the most common cause of community-acquired pneumonia. In the developed world, most pneumococcal deaths occur in the elderly, or in immunodeficient patents including those with sickle cell disease. In the less- developed areas of the world, pneumococcal infection is one of the largest causes of death among children less than 5 years of age (refs. 3, 4, 5, 6). The increase in the frequency of multiple antibiotic resistance among pneumococci and the prohibitive cost of drug treatment in poor countries make the present prospects for control of pneumococcal disease problematical (refs. 7, 8, 9).

The reservoir of pneumococci that infect man is maintained primarily via nasopharyngeal human carriage. Humans acquire pneumococci through aerosols or by direct contact. Pneumococci first colonize the upper airways and can remain in nasal mucosa for weeks or months. As many as 50% or more of young children and the elderly are colonized. In most cases, this colonization results in no apparent infection (refs. 10, 11, 12). Studies of outbreak strains have suggested that even highly virulent strains can colonize without causing disease (refs. 13, 14, 15, 16). These expectations have been recently confirmed using molecular probes to fingerprint individual clones (M. J. Crain, personal communication to one of the inventors). In some individuals, however, the organism carried in the nasopharynx can give rise to symptomatic sinusitis or middle ear infections. If pneumococci are aspirated into the lung, especially with food particles or mucus, they can cause pneumonia. Infections at these sites generally shed some pneumococci into the blood where they can lead to sepsis, especially if they continue to be shed in large numbers from the original focus of infection. Pneumococci in the blood can reach the brain where they can cause meningitis. Although pneumococcal meningitis is less common than other infections caused by these bacteria, it is particularly devastating; some 10% of patients die and greater than 50% of the remainder have life-long neurological sequelae (refs. 17, 18).

In elderly adults, the present 23-valent capsular polysaccharide vaccine is about 60% effective against invasive pneumococcal disease with strains of the capsular types included in the vaccine (refs. 19, 20). The 23-valent vaccine is not effective in children less than 2 years of age because of their inability to make adequate responses to most polysaccharides (refs. 21, 22). Improved vaccines that can protect children and adults against invasive infections with pneumococci would help reduce some of the most deleterious aspects of this disease. A vaccine that protected against disease but did not reduce pneumococcal carriage rates would not, however, be expected to control the disease in immuno-compromised (ref. 20) and in unimmunized individuals. Such a vaccine would also not be expected to affect the rates of infection in immunized children prior to the development of an adequate anti-vaccine response.

A strategy that could control infections in all of these individuals would be any form of immunization that prevented or greatly reduced carriage, and hence transmission of pneumococci. In the case of immunization of young children with Haemophilus influenzae group b polysaccharide-protein conjugates, it has been observed that carriage is reduced from about 4% to less than 1%, (ref. 23), a possible explanation of concomitant herd immunity (ref. 24). If a vaccine could prevent colonization by pneumococci, such vaccine would be expected to prevent virtually all pneumococcal infections in the immunized patients. Since even unimmunized patients must acquire pneumococci from others, a vaccine that reduced carriage should reduce infections in immuno-compromised as well as unimmunized patients. In fact, an aggressive immunization program, coupled with antibiotic treatment of demonstrated carriers, might be able to largely eliminate the human reservoir of this organism. It may not be possible, however, to totally eliminate pneumococci since there are a number of reports that they have been found in laboratory rodents (ref. 25). Whether these pneumococci are infectious for man, easily transmittable to man, or even pathogens in wild rodents is not known. S. pneumoniae does not live free in the environment.

Although intramuscular immunisation with capsular polysaccharide vaccines has been effective at reducing the incidence of pneumococcal sepsis in the elderly (ref. 20), it has not been reported to affect pneumococcal carriage rates in children up to 54 months of age (refs 26, 27). Whether the conjugate vaccine will reduce carriage in children is not known. Thus, the search for a vaccine which can reduce rates of nasopharyngeal carriage must include an examination of non-capsular antigens. Since immunity to carriage would be expected to operate at the mucosal surface, any attempt to identify antigens for vaccines against carriage should include immunisations designed to elicit mucosal immune responses. For these reasons, the present disclosure focuses on intranasal immunisation with pneumococcal proteins in addition to the evaluation of polysaccharide-protein conjugates.

WO-A-94/14318 (Medimmune, Inc.) discloses a method of protecting an animal against S. pneumoniae by administering mycobacteria transformed to encode a protein which elicits antibodies against S. pneumoniae. The protein may be PspA.

In accordance with the present invention, it has now been found that mucosal administration, particularly intranasally, or pneumococcal surface protein A (PspA) or an immunogenic fragment thereof provides protection to a host against pneumococcal colonisation and systemic infection.

Accordingly, in one aspect, the present invention provides an intranasally administrable vaccine composition which confers protection against colonisation by S, pneumoniae in the nasopharynx of a host administered the composition, which comprises:
an effective amount of a pneumococcal surface protein A (PspA) in the form of a killed whole pneumococci, a pneumococcal lysate, an isolated and purified PspA or an immunogenic fragment thereof containing at least one protection-eliciting epitope,
an adjuvanting amount of the B subunit of cholera toxin, and
a pharmaceutical carrier therefor.

The PspA may be in the form of a killed whole S. pneumoniae or a lysate of whole S. pneumoniae. Alternatively, the PspA may be in the form of purified isolated protein or a fragment thereof. Such purified isolated protein or a fragment thereof may be obtained from bacterial isolates or may be formed recombinantly.

In a further aspect, the invention provides a composition comprising pneumococcal surface protein A (PspA) in the form of a killed whole pneumococci, a lysate of pneumococci or an isolated and purified PspA or an immunogenic fragment thereof for preparation of an immunological mucosally administrable composition for use in a method which comprises administering to a host an immunising amount of said substance or composition in conjunction with an adjuvanting amount of the B subunit or cholera toxin (CTB).

The mucosal administration preferably is effected intranasally to provide protection to the host against both pneumococcal colonisation and systemic infection. The intranasal administration also may provide protection to the host against pulmonary infection as well as protection to the host against an infection starting as a pulmonary infection.

In published International patent application WO 92/14488, there are described the DNA sequences for the pspA gene from S. pneumoniae Rx1, the production of a truncated form of PspA by genetic engineering and the demonstration that such truncated form of PspA confers protection in mice to challenge with live pneumococci.

From sequences of the pspA gene, it has been shown that PspA proteins are variable in size (roughly 70kDa). The C-terminal 37% of the molecule is largely composed of the 20-amino acid repeats which form a binding site that permits PspA to attach to the phosphocholine residues of the pneumococcal lipoteichoic acids. The central region of PspA is rich in proteins and is suspected to be the portion of the molecule that passes through the cell wall. The sequence of the N-terminal 80% of the molecule is largely a-helical and contains the region of PspA that can elicit antibodies that are protective against sepsis. Although PspA's are almost always at least slightly different from one another, there is enough cross-reactivity between them that antibodies to one PspA detect PspAs on all pneumococci. Moreover, immunization with one PspA can either protect against death or delay death with virtually all different challenge strains. Accordingly, a mixture of a small number of PspAs could elicit effective immunity against most pneumococci.

The immunoprotective truncated PspAs described in WO 92/14488 may be used in the present invention as the PspA fragments described above for mucosal administration.

The ability of a vaccine to protect against pneumococcal colonization, as provided herein, means that the active component may protect against disease not only in the immunized host but, by eliminating carriage among immunized individuals, the pathogen and hence any disease it causes may be eliminated from the population as a whole.

In the data presented herein, it is shown that intranasal administration can also prevent sepsis resulting from intratracheal administration of pneumococci, so that the vaccine can protect against both pneumococcal colonization and sepsis.

The principal determinant of specific immunity at mucosal surfaces is secretory IgA (S-IgA) which is physiologically and functionally separate from the components of the circulatory immune system. S-IgA antibody responses may be induced locally by the application of suitable immunogens to a particular mucosal site. The bulk of mucosal S-IgA responses, however, are the results of immunity generated via the common mucosal immune system (CMIS) (ref. 28), in which immunogens are taken up by specialized lympho-epithelial structures, collectively referred to as mucosa-associated lymphoid tissue (MALT). The best studied immunologic lympho-epithelial structures are the gut-associated lymphoid tissues (GALT), such as intestinal Peyer's patches. It is now clear, however, that other structurally and functionally similar lymphoid follicles occur at other mucosal surfaces, including those of the respiratory tract (ref. 29).

Bronchus-associated lymphoid tissue (BALT) was described by Bienenstock (refs. 30, 31) in experimental animals, but is apparently not present in the noninfected human bronchial tree (ref. 32). The upper respiratory tract in humans, however, is furnished with Waldeyer's ring of tonsils and adenoids. In rodents, the functional equivalent of these consists of nasal-associated lymphoid tissue (NALT), a bilateral strip of lymphoid tissue with overlying M-like epithelial cells at the base of the nasal passages (ref. 33).

In the experimental results set forth in the Examples below, it is shown that mice can be effectively immunized by intranasal (i.n.) instillation of bacterial protein immunogens, particularly when conjugated to or mixed with cholera toxin (CT) or its B subunit (CTB) (ref. 34). When CTB is used as an adjuvant for i.n. immunizations, specific IgA antibodies are induced in secretions of the intestinal, respiratory, and genital tracts, as well as predominantly IgA antibody-secreting cells in the intestinal lamina propria and salivary glands. Strong circulatory immune responses are also induced, with IgG and IgA antibodies in the serum, and IgG and IgA antibody-secreting cells in the spleen. The circulatory (or systemic) immune responses elicited by i.n. administration of antigens along with CTB are comparable with, or even stronger than, those induced by the administration of similar immunogens by the intragastric (i.g.; peroral) route (refs. 34, 35). Accordingly, it appears that i.n. immunization is an effective route for stimulating common mucosal responses as well as circulatory antibody responses and requires less antigen than i.g. immunization.

Most soluble or non-replicating antigens are poor mucosal immunogens, especially by the peroral route, probably because they are degraded by digestive enzymes and have little or no tropism for the GALT. A notable exception is CT, which is a potent mucosal immunogen (ref. 36), probably because of the G_{M1} ganglioside-binding property of its binding subunit, CTB, that enables it to be taken up by the M cells of Peyer's patches and passed to the underlying immunocompetent cells. In addition to being a good mucosal immunogen, CT is a powerful adjuvant (refs. 37, 38, 39). When administered in µg doses, CT greatly enhances the mucosal immunogenicity of other soluble antigens co-administered with it.

In the experimental results contained in the Examples below, it is shown that CTB is a strong adjuvant when given i.n. in mice along with the pneumococcal protein, PspA. Although the inventors cannot completely rule out a role for small amounts of CT in these studies, CT was <0.1% of the 5 µg dose of CTB that was administered. Thus, it would appear that when administered i.n., CTB may be a stronger adjuvant and act more independently of CT, than when it is given i.g. For example, when given orally or i.g., CTB has no direct adjuvant effect, but can act synergistically with CT (ref. 40).

The mechanisms by which CT and CTB act as adjuvants are not fully understood, but are certainly complex, and appear to depend on several factors, including: 1) the toxic activity associated with the ADP-ribosylating property of the A1 subunit (ref. 41); 2) increased permeability of mucosae (refs. 42, 43), 3) enhanced antigen-presenting cell function (with increased levels of IL-1) (refs. 44, 45), as well as 4) direct stimulation of T and B cell activities (refs. 46, 47, 48, 49). This last point is controversial, however, as the in vitro effects of CT or CTB on T and B cells are generally inhibitory rather than stimulatory (refs. 50, 51, 52). Nevertheless, numerous reports attest to the in vivo mucosal immunoenhancing effects of CT and of CTB coupled to antigens (refs. 38, 39, 53, 54, 55, 56).

Although carriage of pneumococci can be maintained for long periods in the very young and the elderly, it is generally not a permanent condition. Carriage is much less common in older children and young adults (refs. 10, 11, 12, 57, 58). One explanation for these findings is that carriage may be interfered with by immunity (possibly mucosal immunity) to pneumococci (refs. 11, 59). The inventors have shown that most human saliva have IgA antibodies to type 23 capsular polysaccharide and phosphocholine (an immunodominant determinant of pneumococcal cell wall teichoic acids (ref. 60)). It seems likely, therefore, that human sera would also contain antibodies to other pneumococcal antigens. In the case of group A streptococci, M proteins have been shown to be required for colonization in rats, and antibodies to M proteins can protect against colonization of the throat (refs. 61, 62). In mice, the inventors have shown herein that antibody to PspA can prevent carriage of S. pneumoniae.

Antibodies may be effective against carriage in two ways, namely: 1) they might act at the mucosal surface by opsonizing pneumococci, preventing attachment or surface invasion; 2) they might act via opsonophagocytosis and killing. This latter mechanism could be especially important if nasopharyngeal carriage is dependent on minimal invasion of the nasal mucosal surface. The complement fixing antibodies could prevent the invasion and facilitate the killing of any pneumococci that invaded locally. Alternatively, complement fixing antibodies might be able to act and the mucosal surface if inflammation causes a sufficient release of complement, phagocytes, and possibly serum antibody.

One of these mechanisms might play a role in the observation that carriage of H. influenzae can be prevented by an intramuscular vaccine (ref. 23). It has recently been reported that significant levels of H. influenzae polysaccharide-specific IgG and IgA are detected in secretions of children following immunization with the group b polysaccharide conjugate vaccine (refs. 24, 63).

Although definitive comparisons have not been made in most cases, existing mouse protection data (refs. 64, 65, 66, 67) suggests that antibodies that can opsonize pneumococci (e.g. those to the capsule) are generally more protective against sepsis than those that block the activities of toxins (e.g. pneumolysin) or enzymes (e.g. autolysin or neuraminidase). However, at the mucosal surface, the role played by antibodies that inactivate toxins and enzymes may be greater than that played by opsonic antibodies. The reason to suspect this is that for opsonic antibodies to exert their anti-bacterial effect, complement and phagocytes are required. Phagocytes are rare on the surface of normal nasopharyngeal tissue, and even if present, the phagocytes do not have the filtering action of the spleen and reticuloendothelial system to increase their chance of interactions with opsonized bacteria. Antibodies that block the virulence enhancing effects of pneumolysin and pneumococcal enzymes should able to bind their antigens just as effectively whether phagocytes were present or not.

The results provided herein show that i.n. immunization with heat-killed pneumococci, and pneumococcal lysates, and purified PspA can protect mice against nasopharyngeal carriage. As noted earlier, the ability of a vaccine to protect against colonization means that it may protect against disease not only in the immunized host, but, by eliminating carriage among immunized individuals, the pathogen and hence any disease it causes may be eliminated from the population as a whole.

The vaccine composition which is administered intranasally as provided herein may be formulated in any convenient manner and in a dosage formulation consistent with the mode of administration and the elicitation of a protective response. The quantity of antigen to be administered depends on the subject to be immunized and the form of the antigen. Precise amounts and form of the antigen to be administered depend on the judgement of the practitioner. However, suitable dosage ranges are readily determinable by those skilled in the art and may be of the order of micrograms to milligrams. Suitable regimes for initial administration and booster doses also are variable, but may include on initial administration followed by subsequent administrations.

### EXAMPLES

### Example 1

This Example illustrates the provision of a model for pneumococcal carriage in mice.

Seven different strains of S. pneumoniae were inoculated in 10 µl volumes with log-phase cultures in the nares of CBA/N XID mice over a period of several minutes using slow delivery from a 20 µl Pipetman. After 6 to 7 days, the mice were sacrificed and their trachea was cut just below the larynx. 50 µl of sterile saline was instilled and washed out through the nares. The area washed represents the pharynx and nasal tissues. Six of these strains was able to establish carriage without concomitant sepsis or bacteremia. One of the seven strains, (A66, a virulent capsular type 3 strains) killed all four of the i.n. infected CBA/N mice within 3 days. The results are shown in the following Table I:

**TABLE I**

| Carriage of *S. pneumoniae* in CBA/N (XID) mice: 10⁷ CFU i.n. | | | | | | |
|---|---|---|---|---|---|---|
| Strain | Capsular type | Log LD₅₀ i.v. | Alive: Dead | % With Carriage | Median CFU in nose | Max. CFU/50 µl blood |
| A66 | 3 | <2 | 0:4 | -- | -- | -- |
| BG9739 | 4 | <2 | 3:1^{a} | 67^{a} | 1,000 | <3 |
| L82106 | 6B | ≥ 7 | 6:0 | 100 | 2,000 | <3 |
| BG9163 | 6B | 3.5 | 2:0 | 100 | 2,000 | <3 |
| TJ0893 | 14 | .4 | 4:0 | 100 | 4,000 | <3 |
| L82013 | 19 | ≥ 2 | 4:0 | 100 | 200 | <3 |
| BG8826 | 23F | ≥ 2 | 2:0 | 100 | 20,000 | <3 |
| Note: Carriage and blood CFU determined after 7 days. Greater than 10 CFU of pneumococci were recovered from the nose of all mice judged as carriers. | | | | | | |

| | | | | | | |
|---|---|---|---|---|---|---|
| ^{a} One mouse in this group that grew out a contaminant in large numbers and no pneumococci. It seems likely that the contaminant excluded the pneumococci. | | | | | | |

### Example 2:

The procedure of Example 1 was repeated by inoculating the nares of BARB/By mice with two of three pneumococcal isolates used. Carriage was observed with all three strains, without bacteremia or sepsis. The results are shown in the following Table II:

**TABLE II**

| Carriage of *Streptococcus pneumoniae* isolates in BALB/ByJ mice | | | | | | |
|---|---|---|---|---|---|---|
| Strain | Capsular type | Log LD₅₀ i.v. | Alive: Dead | % With Carriage | Median CFU in nose | Max. CFU in 50µl of blood |
| D39 | 2 | 7 | 4:0 | 100 | 400 | <3 |
| A66 | 3 | 4 | 4:0 | 100 | 4,000 | <3 |
| BG7322 | 6B | -7 | 4:0 | 75 | 1,700 | <3 |
| L82013 | 19 | ≥8 | 4:0 | 100 | 6,000 | <3 |
| BG8826 | 23F | ≥8 | 4:0 | 75 | 1,900 | <3 |
| Note: Carriage and blood CFU determined after 7 days. Note: CFU in nose expressed as CFU in 50µl of 1ml nasal wash | | | | | | |

### Example 3:

This Example shows the effects of i.n. inoculation on lung infection.

With three of the strains examined for carriage in Example 1, the numbers of pneumococci in the lung were examined at the time of sacrifice. The results obtained are shown in the following Table III:

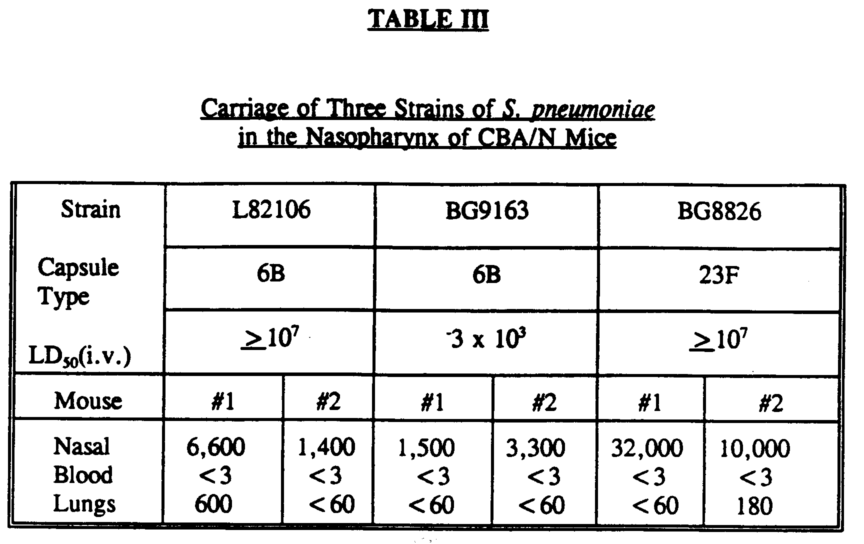

The data shown in this Table III illustrates the fact that, although the carried pneumococci do not generally cause detectable bacteremia or sepsis, they can spread in at least small numbers to the lung. The ability of S. pneumoniae to spread from the nasopharynx to the lung emphasizes the importance of a vaccine blocking carriage in the upper airways.

### Example 4

This Example shows the effect of i.n. inoculation on colonization stability.

Colonization appeared to be present for at least 19 days and stable for at least 6 days, as shown by the results in the following Table IV:

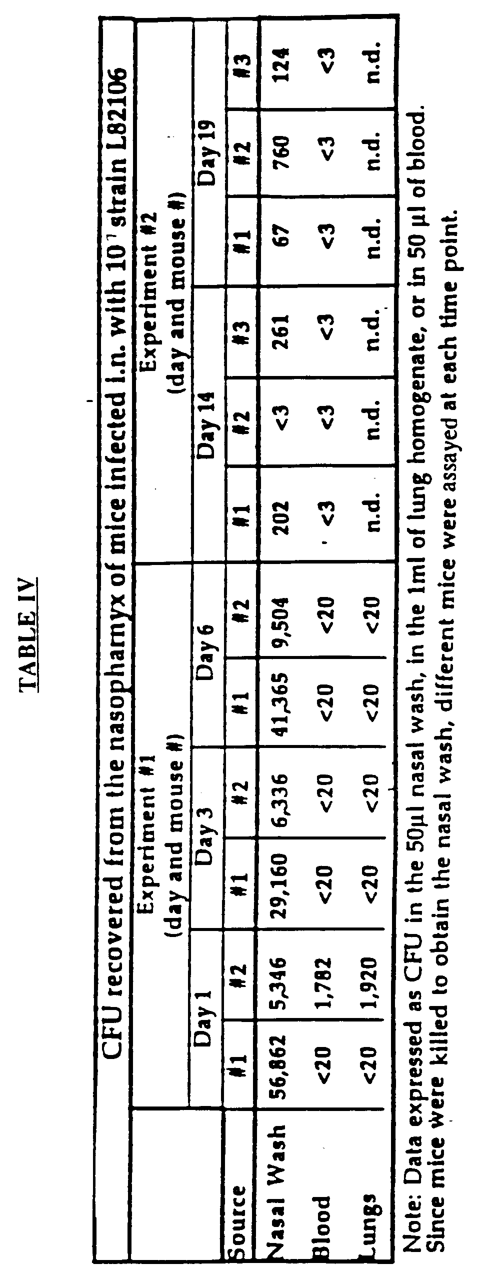

To identify the pneumococci from nasal washes in the experiments reported in Examples 1 to 4, they were plated on gentamicin plates since this antibiotic does not kill pneumococci but kills most other bacterial from the nose (ref. 68). Individual colonies from each nasal wash were then picked and replated with an optochin disk to confirm that they were pneumococci. In some cases, the bacteria were capsule-typed to be sure that they were, in fact, the same bacteria with which inoculated the mice were inoculated. Control mice that received no bacteria yielded no bacteria that grew on 0.02% gentamicin and were sensitive to optochin. Subsequent studies have shown that challenge with as few as 2x10⁷ colony forming units (CFU) also yields carriage in all mice. Lower doses down to 10³ CFU yield comparable carriage in most mice but as many as 1/4 to 1/3 of the mice fail to carry any pneumococci after 1 week. These studies indicate that the ideal dose is probably between 10⁷ and 10⁶ CFU of L81905.

### Example 5

This Example illustrates elicitation of protection against carriage by immunization with heat killed pneumococci and with a lysate of pneumococci.

CBA/N mice were immunized i.n. with 2 x 10⁷ heat-killed (60°C) L82016 or a lysate of an equal number of L82016. Mice were given three i.n. immunizations spaced 10 days apart. The first two injections were given with 5 µg of CTB. Two weeks after the last injection the mice were challenged with 10⁸ CFU of live L82016. The results obtained are set forth in the following Table V:

**TABLE V**

| Elicitation of protection against carriage by immunisation with heat killed and autolysed pneumococci | | | | |
|---|---|---|---|---|
| Immunogen | CFU from individual mice | Geometric mean CFU | P vs CTB | P vs none |
| Heat killed L82016 + CTB | <3, <3, 3, 9, 26 | 4.8 | <.01 | <.02 |
| Autolysed L82016 + CTB | <3, <3, <3, 8, 30 | 4.5 | <.01 | <.02 |
| CTB | 9,160,197,248,741 | 139 | n.s. | n.s. |
| None | 6,1340,>1400,>1400 | >354 | n.s. | n.s. |
| P. values calculated by Student's t-test. n.s. = not significantly different | | | | |

### Example 6

This Example illustrates protection against carriage by immunisation with isolated and purified PspA.

CBA/N mice at 10 weeks of age were immunised i.n. with PspA purified by passage over a choline-Sepharose column and eluted in 2% choline chloride. The material run over the column is clarified medium from a pneumococcal culture grown to late log phase in a defined medium (Rijn et al, Infect. Immun. 1990, 27, pp 444-448), containing 0.03% ethanolamine and no more than 0.00,001% choline chloride. When pneumococci is grown in this medium, about half of the PspA is released from the cell surface and can be recovered in the medium. Most pneumococci can not grow in this medium and must be adapted by growing them in CDM ("chemically defined medium" - Rijn et al, Infect. & Immun. 1990, Vol. 27, pp 444-448) with successive lower concentrations of choline.

The PspA eluted from the column comprises at least 99% of the eluted protein.

The CBA/N mice were immunised i.n. with 150 ng or s.c. with 1 µg of ≥99.9% pure PspA from strain L82016.

Three i.n. immunizations were given at 10 day intervals. 5 µg of commercially-obtained ≥99.9% pure (OTB) (List Biochemical Labs, Inc., Campbell, CA) were given with the first two injections as an adjuvant. In the case of s.c. immunization, the first injection was given with PspA in Freund's complete adjuvant and a second injection of PspA in saline was given two weeks later. Two weeks after the last injections, the mice were inoculated i.n. with 10⁷ CFU of L82016. Seven days later, the mice were sacrificed, bled and assayed for carriage in the nasopharynx. The results obtained are illustrated in the following Table VI:

**TABLE VI**

| Intranasal immunization with isolated PspA elicits protection against carriage in CBA/N mice challenged i.n. with 10⁷ CFU of L82016 | | | | |
|---|---|---|---|---|
| Immunization | | Carriage | | |
| Immunogen | Route | Yes: No (≤3 CFU: ≤3 CFU) | Log mean CFU (S.E. PspA factor) | P vs. + CTB |
| PspA | i.n. | 4:0 | 440 (x/+2.6) | 0.014 |
| PspA + CTB | i.n. | 0:4 | <3 | -- |
| CTB | i.n. | 8:0 | 440 (x/+2/2) | 0.002 |
| PspA + CFA | s.c. | 4:0 | 240 (x/+1/8) | 0.014 |
| CFA | s.c. | 4:0 | 190 (x/+1.6) | 0.014 |
| None | None | 4:0 | 1260 (x/+4.4) | 0.014 |
| P values calculated by Fisher exact test. A one way ANOVA gave a P value of 0.02. | | | | |

None of the mice exhibited detectable pneumococci in their blood at the time of assay. As may be seen from Table VI, carriage was seen in all mice except the group of four immunized in. with PspA and CTB. Control mice immunized with CTB or PspA alone still exhibited carriage.

In another experiment mice were infected i.n. with ten times the dose (10⁸ CFU), and immunization with PspA + CTB still protected against pneumococcal carriage (Table VII below). In that experiment the control mice were injected with CTB plus a comparable preparation from a PspA- strain WG44.1, made by the identical purification procedures used for PspA. Mice were immunized with a dilution of the WG44.1 material comparable to that of the isolated PspA. The failure of these control mice to be protected against carriage makes it clear that the protection elicited by isolated PspA is due to PspA and not an undetected contaminant co-isolated with PspA.

When mice were immunized s.c., although they produced humoral antibody to PspA, they still exhibited carriage.

**TABLE VII**

| Intranasal immunization with isolated PspA elicits protection against carriage in CBA/N mice challenged i.n. with 10⁸ CFU of L82016 | | | | |
|---|---|---|---|---|
| Immunogen | CFU from individual mice | Geometric mean CFU | P vs PapA- | P vs pooled controls |
| FL-L82016 PspA + CTB | <3, <3, <3 | <3 | 0.028 | <0.0001 |
| PspA-(WG44.1) + CTB | 128, 18, 277, 49, 527 | 4,875 | - | - |
| CTB | 1,059, 26,720, dead¹ | 11,226 | - | - |
| None | 426, 11,484, dead¹ | 6154 | - | - |
| P. values calculated by Welch's t-test. n.s. = not significantly different. | | | | |

| | | | | |
|---|---|---|---|---|
| ¹ for the purpose of the statistical calculations, these two mice were assigned carriage values of 50,000 since 49,000 was the highest carriage level observed in a live animal. | | | | |

### Example 7

This Example illustrates the elicitation of secretory and systemic immune responses by i.n. immunization with PspA.

Using the immunization protocol of Example 6, further immunization studies were carried out and mice were bled 10 days after immunization. Secretory and systemic antibody responses were determined and the results appear in the following Table VIII:

**TABLE VIII**

| Antibody to PspA elicited by s.c. and i.t. immunization with PspA | | | | | |
|---|---|---|---|---|---|
| Antigen | Route | IgM µG | Serum IgG µg | Salivary IgG % specific | IgA % specific |
| PspA + CTB | i.n. | < 1.5 | 33 ± 14 | 8.7 ± 3 | 13.5 ± 2.6 |
| PspA | i.n. | < 1.5 | < 1.5 | n.d. | n.d. |
| WG44.1 + CTB | i.n. | < 1.5 | < 1.5 | < 2 | < 2 |
| CTB | i.n. | < 1.5 | < 1.5 | < 2 | < 2 |
| PspA + CFA | s.c. | < 1.5 | 470 ± 217 | n.d. | < 2 |
| CFA | s.c. | < 1.5 | 1.5 | n.d. | < 2 |
| Note: Salivary responses are expressed as percent of total immunoglobulin that is specific to PspA. | | | | | |

As may be seen from this Table, the immunization elicited detectable salivary IgG and IgA antibody responses. Control immunizations with CTB, CFA or a PspA⁻fraction isolated from PspA⁻ strain WG44.1 did not elicit antibody to PspA. Antibody was not detected when CTB was not used as an adjuvant for i.n. immunization. Assays for antibody to PspA were conducted with ELISA plates coated with isolated PspA.

### Example 8

This Example illustrates elicitation of cross-protection against carriage with strains whose PspAs differ from those of immunizing PspA R36A.

Mice were immunized with three i.n. immunizations with 0.15 µg of R36A PspA. In the first two injections immunization was accompanied with 4µg of purified CTB. Injections were 10 days apart and mice were challenged about 2 weeks after the last immunization. The control mice received the two CTB immunizations and were "immunized" with saline only for the third injection. These mice were largely protected from challenge carriage with two different challenge strains BG7322 and BG8826 of different capsular types and different PspA types. Both have PspA of a different type than the serotype 25 PspA used for immunization. The R36A PspA immunization came from strain R36A which is a non-encapsulated mutant of capsular type 2 strain D39. Thus, neither the capsular type nor the PspA type of the strains providing the immunizing PspA were the same as the challenge strains. Because of the small number of mice in each group the results with the individual strains are not quite significant. However, pooling the data lead to a highly significant demonstration that PspA can elicit protection against challenge pneumococci with a PspA different from the immunizing PspA. The data is summarized in the following Table IX:

**Table IX**

| **Intranasal immunization with isolated R36A PspA (PspA type 25) elicits cross-protection against carriage in CBA/N challenged i.n. with 10**^{**7**} **CFU of strains BG7322 (PspA type 24) and BG8826 (PspA type 20)** | | | | | | | |
|---|---|---|---|---|---|---|---|
| *Streptococcus pneumoniae* Challenge Strain | | | Immunized with | CFU recovered from nasopharynx | | *P* versus CTB only | Max. CFU /50µl of blood |
| Name | Caps. type | PspA type | | individual mice | median | | |
| BG7322 | 6B | 24 | PapA + CTB | <3, <3, <3, 12 | <3 | 0.057 | <3 |
| | | | CTB | <3, 1500, 1800, 4000 | 1700 | | <3 |
| BG8826 | 23F | 20 | PspA + CTB | <3, <3, <3, 3 | <3 | 0.057 | <3 |
| | | | CTB | <3, 29, 3700, 5800 | 1900 | | <3 |
| BG7322 + BG8826 | encapaulated | not 25 | PspA + CTB | <3, ,3, <3, <3, <3, <3, 3,12 | <3 | 0.013 | <3 |
| | | | CTB | <3, <3, 29, 1500, 1900, 3700, 4000, 5800 | 1700 | | <3 |
| *P* value calculated by the Wilcoxon two sample rank test using one degree of freedom. **Note:** CPU in nose expressed as CPU in 50µl of 1 ml nasal wash. | | | | | | | |

### Example 9

This Example illustrates elicitation of protection against intratracheal (i.t.) challenge by intranasal immunization with PspA.

Whole-length PspA was recovered from S. pneumoniae R36A strain (which provides the same PspA as the Rxl strain). The strain was grown in 100 µl chemically-defined medium (Rijn et al, Infect. & Immun. 1990, vol. 27, pp 444-448), except that the medium contained 0.03% choline chloride. The bacteria were harvested in late log phase (about 5x10⁷ CFU/ml) and washed five times with 20 ml of saline followed by centrifugation at 2000xg for 10 minutes. With each wash, the bacteria was saved and the supernatant discarded. The washed cells then were eluted with 5 ml of 2% choline chloride and the eluted material was shown to contain PspA by dot blot using monoclonal antibody XiR 278.

An identical procedure was carried out on the S. pneumoniae strain WG44.1 (McDaniel et al (III)), which does not produce PspA because of the absence of an upstream portion of the pspA gene. This material provided a control in that the preparation should contain the same general impurities that might be in the extract from R36A. The material recovered from the washed WG44.1 cells by elution with 2% choline chloride did not contain detectable PspA by dot blot, as expected.

For the purpose of administration, the PspA preparation from R36A was diluted 1:2. 12 µl of the solution contained 5 µg of added cholera toxin B subunit (CTB) as an adjuvant and was instilled into the nose of each BALB/cJ mouse. Thirty-two and forty-two days later the immunization was repeated in an identical manner. As a control, some mice were immunized with an identical preparation from the WG44.1 S.pneumoniae strain. A final group was left unimmunized. Seven days after the third dose, the immunized mice were challenged with 2x10⁶ CFU (100xLD₅₀) of A66 S. pneumoniae intratracheally.

Activity assays also were performed on sera obtained seven days after the third immunization on separate groups of mice immunized by protocols almost identical to those noted above.

The results obtained are set forth in the following Tables X and XI:

**TABLE X**

| Protection against challenge with 10⁶ A66 after i.n. or s.c. immunization with 150 µg doses of PspA | | | | | |
|---|---|---|---|---|---|
| Antigen | Immunization Route | Challenge Route | % Alive | Median day of death | P value vs. matched control |
| PspA + CTB | i.n. | i.t. | 100 | > 16 | < 0.0001 |
| PspA only | i.n. | i.t. | 25 | 4 | |
| PspA- + CTB | i.n. | i.t. | 11 | 4 | |
| CTB | in. | i.t. | 0 | 4 | |
| None | -- | i.t. | 0 | 4 | |
| PspA + CFA | s.c. | i.t. | 80 | > 16 | 0.02 |
| CFA | s.c. | i.t. | 0 | 4 | |
| P values calculated by the Fisher exact test. | | | | | |

**TABLE XI**

| Intranasal Immunization with Rx1 PspA and Intratracheal Challenge with Capsular Type 3 Strain A66 | | | | | |
|---|---|---|---|---|---|
| Immunogen | Adjuvant | IgG anti-PspA (µg/ml) | Challenge CPU of A66 | CFU/ml at day 3* | Day of* Death |
| FL PspA (R36A) | CTB | 17.8 ±3.3 | 2 x 10⁶ | < 10² | > 12** |
| | " | | " | < 10² | > 12 |
| | " | | " | < 10² | > 12 |
| | " | | " | < 10² | > 12 |
| PspA⁻ (WG44.1) | CTB | ≤0.4 | 2 x 10⁶ | 2 x 10⁸ | 3 |
| | " | | " | 4 x 10⁵ | 4 |
| | " | | " | 4 x 10⁴ | 4 |
| | " | | " | 2 x 10⁴ | 4 |
| Saline | Saline | ≤0.4 | 2 x 10⁶ | N.D. | 4 |
| " | " | | " | N.D. | 4 |
| " | " | | 2 x 10⁵ | N.D. | 4 |
| " | " | | " | N.D. | 5 |

| | | | | | |
|---|---|---|---|---|---|
| ** FL-PspA vs. Saline (or WG44. 1) at P <.005 | | | | | |
| * Results with individual mice | | | | | |

As can be seen in Table X, 24 hours after infection the unimmunized and the mock immunized mice had high levels of pneumococci in their blood. These mice all died on day 3 post challenge. The mice immunized with PspA exhibited no detectable pneumococci on day 3 and all survived infection. Although the data clearly indicate that i.n. immunization with PspA can protect against pulmonary challenge, the strains of S. Pneumoniae used, survives well in blood and causes rapid death in mice when injected i.v. (LD₅₀ < 10²). Thus, the protection against death may have been due to protection against sepsis as well as pulmonary infection protection had been elicited in the lung.

### Example 10

Mucosal immunization with R36A PspA can provide protection against intratracheal and systemic (intravenous) challenge with capsular type 3 A66 Streptococcus pneumoniae.

BALB mice were immunized i.n. three times with 150ng of PspA at 10 day intervals. With each injection they also received 5µg of purified CTB. The last immunization was given in saline. Control mice received the two CTB injections but no PspA. Four weeks after the last injection the mice were challenged with strain A66 type 3 S. pneumoniae by the i.t. or i.v. route at the doses indicated in Table XII. We observed that i.n. immunization could protect against systemic infections by both the i.t. and i.v. route of challenge. In the case of the i.t. route, the immunization protected against the fatal effects of pulmonary infections and probably also sepsis. In the case of the i.v. infection where the pneumococci are injected directly into the blood, the immunization protected against sepsis.

**TABLE XII**

| Intranasal immunization with R36A PspA can protect against systemic infection with *S. pneumoniae*. | | | | | | | |
|---|---|---|---|---|---|---|---|
| Challenge | | Immunogen | Days to Death | | | Survival | |
| Route | Log Dose | | Individual | Median | P vs. control¹ | Alive: Dead | *P* vs. control² |
| i.t. | 5.1 | PspA + CTB | >21, >21, >21, >21, >21 | >21 | | 5:0 | |
| | | CTB | 3, 3, 4, 4, 4, 5, 6, >21, >21, >21 | 4.5 | 0.02 | 3:7 | 0.02 |
| i.v. | 4.9 | PspA + CTB | >21, >21, >21, >21, >21 | >21 | 0.045 | 5:0 | 0.042 |
| | | CTB | 4, 4, 4, 4, 5, 5, >21, >21, >21, >21 | 5 | | 4:6 | 6 |

| | | | | | | | |
|---|---|---|---|---|---|---|---|
| ¹ calculated by one tailed Wilcoxon two sample rank test. | | | | | | | |
| ² calculated by one tailed Fisher exact test. | | | | | | | |

In summary of this disclosure, the present invention provides a method of preventing colonization of pneumococci in a host and for protecting against systemic infection by pneumococci, by mucosal, particularly intranasal (intranasopharyngeal), administration of PspA in various forms. Modifications are possible within the scope of the invention.

### REFERENCES

1. Anonymous. Centers for Disease Control HIV/AIDS Serveillance Report. 1991; August :1-18.
2. Fraser DW. What are our bacterial disease problems. In: JB Robbins, Hill JC, Sadoff JC ed. Bacterial Vaccines. New York: 1982: xix-xxiv.
3. Berman S, McIntosh K. Selective primary health care: stratagies for control of disease in the developing world. XXI acute respiratory infections. Rev. Infect. Dis. 1985; 7 :647-491.
4. Greenwood BM, Greenwood AM, Bradley AK, Tulloch S, Hayes R, Oldfield FSJ. Deaths in infancy and early childhood in a well vaccinated, rural, West African population. Ann. Trop. Pediatr. 1987; 7 :91-99.
5. Spika JS, Munshi MH, Wojtyaniak B, Sack DA, Hossain A, Rahman M, Saha SK. Acute lower respiratory infections: a major cause of death in children in Bangladesh. Ann. Trop. Pediatr. 1989; 9 :33-39.
6. Bale JR. Etiology and epidemiology of acute respiratory tract infections in children in developing countries. Rev. Infect. Dis. 1990; 12 (Suppl 8) :S861-S1083.
7. Munoz R, Musser JM, Crain M, Briles DE, Marton A, Parkinson AJ, Sorensen U, Tomasz A. Geographic distribution of penicillin-resistant clones of *Streptococcus pneumoniae*: characterization by penicillin-binding protein profile, surface protein A typing, and multilocus enzyme analysis. Clinic. Infect. Dis. 1992; 15 :112-118.
8. Marton A, Gulyas M, Munoz R, Tomasz A. Extremely high incidence of antibiotic resistance in clinical isolates of Streptococcus pneumoniae in Hungary. J. Infect. Dis. 1991; 163 :542-548.
9. Klugman KP. Pneumococcal resistance to antibiotics. Clin. Microbiol. Rev. 1990;
10. Gray BM, Converse GM III, Dillon HC. Epidemiologic studies of *Streptococcus pneumoniae* in infants: acqusition, carriage, and infection during the first 24 months of life. J. Infect. Dis. 1980; 142 :923-933.
11. Gray BM, Converse GM III, Huhta N, Johnston RB Jr., Pichichero ME, Schiffman G, Dillon HC Jr. Antibody response to pneumococcal carriage. J. Infect. Dis. 1981; 142 :312-318.
12. Hendley JO, Sande MA, Stewart PM, al. e. Spread of Stereptococcus pneumoniae in families. I. Carriage rates and distribution of types. J. Infect. Dis. 1975; 132 :55.
13. Smillie WG, Warnock GH, White HJ. A study of a type I pneumococcus epidemic at state hospital at Worchester Massachusettes. Am J Pub Hlth 1938; 28 :293-302.
14. Smillie WG. A study of an outbreak of type II pneumococcal pneumonia in the Veterans Administration Hospital at Bedford, Massachusetts. Am. J. Hyg. 1936; 24 :522-535.
15. Gratten M, Naraqi S, Hansman D. High prevalence of penicillin-insensitive penumococci in port moresby, Paupa New Guinea. Lancet 1980; ii :192-195.
16. DeMaria TF, McGhee RB, Lim DJ. Rheumatoid factor in otitis media with effusion. Arch. Otolaryngol. 1984; 110 :279-280.
17. Bohr V, Rasmussen N, Hansen B, Gade A, Kjersem H, Johsen N, Paulson O. Pheumococcal meningitis: An evaluation of prognostic factors in 164 cases based on mortality and on a study of lasting sequelae. J. Infect. Dis. 1985; 10 :143-157.
18. Klein JO. The epidemiology of pneumococcal diseases in infants and children. Rev. Infect. Dis. 1981; 3 :246-.
19. Bolan G, Broome CV, Facklam RR, Plikaytis BD, Fraser WD, Schlech WFI. Pneumococcal vaccine efficacy in selected populations in the Unites States. Ann. Intern. Med. 1986; 104 :1-6.
20. Shapiro ED, Berg AT, Austrian R, Schroeder D, Parcells V, Margolis A, Adair RK, Clemmens JD. Protective efficacy of polyvalent pneumococcal polysaccharide vaccine. N. Engl. J. Med 1991; 325 :1453-1460.
21. Cowan MJ, Ammann AJ, Wara DW, Howie VM, Schultz L, Doyle N, Kaplan M. Pneumococcal polysaccharide immunization in infants and children. Pediatrics 1978; 62 :721-727.
22. Gotschlich EC, Goldschneider I, Lepow ML, Gold R. The immune response to bacterial polysaccharides in man. Antibodies in human diagnosis and therapy. . New York: Raven, 1977: 391-402.
23. Barbour ML, Mayon-White RT, Crook DW, Coles C, Moxon ER. The influence of *Haemophilus influenzae* type b (Hib) conjugate vaccine (PRP-T) on oropharyngeal carriage of Hib in infants under 12 months of age. ICAAC Abstracts 1993; 33 :175.
24. Chiu SS, Greenberg PD, Marcy SM, Wong VK, Chang SJ, Chiu CY, Ward JI. Mucosal antibody responses in infants following immunization with *Haemophilus influenzae*. Pediatric Res. Abstracts 1994; 35 :10A.
25. Fallon MT, Reinhard MK, Gray BM, Davis TW, Lindsey JR. Inapparent *Streptococcus pneumoniae* type 35 infections in commercial rats and mice. Laboratory Animal Science 1988; 38 :129-.
26. Douglas RM, D H, Miles HB, Paton JC. Pneumococcal carriage and type-specific antibody Failure of a 14-valent vaccine to reduce carriage in healthy children. American Journal of Diseases of Children 1986; 140 :1183-1185.
27. Douglas RM, Miles HB. Vaccination against *Streptococcus pneumoniae* in childhood: lack of demonstrable benefit in young Australian children. Journal of Infectious Diseases 1984; 149 :861-869.
28. Mestecky J. The common mucosal immune system and current strategies for induction of immune response in external secretions. J. Clin. Immunol. 1987; 7 :265-276.
29. Croitoru K, Bienenstock J. Characteristics and functions of mucosa-associated lymphoid tissue. In: PL Ogra, Mestecky J, Lamm ME, Strober W, McGhee JR, Bienenstock J ed. Handbook of Mucosal Immunology. San Diego, CA: Academic Press, Inc., 1994: 141-149.
30. Bienenstock J, Johnston N, Perey DY. Bronchial lymphoid tissue. I. Morphologic characteristics. Lab. Invest. 1973; 28 :686-692.
31. Bienenstock J, Johnston N, Perey DY. Bronchial lymphoid tissue. II. Functional characterisitics. Lab. Invest. 1973; 28 :693-698.
32. Pabst R. Is BALT a major component of the human lung immune system? Immunology Today 1992; 13 :119-122.
33. Kuper CF, Koornstra PJ, Hameleers DMH, Biewenga J, Spit BJ, Duijvestijn AM, van Breda Vriesman PJC, Sminia T. The role of nasopharyngeal lymphoid tissue. Immunol. Today 1992; 13 :219-224.
34. Wu H-Y, Russell MW. Induction of mucosal immunity by intranasal application of a streptococcal surface protein antigen with the cholera toxin B subunit. Infection and Immunity 1993; 61 :314-322.
35. Russell MW, Wu H-Y. Distribution, persistence, and recall of serum and salivary antibody responses to peroral immunization with protein antigen I/II of *Streptococcus mutans* coupled to the cholera toxin B subunit. Infection and Immunity 1991; 59 :4061-4070.
36. Elson CO, Ealding W. Generalized systemic and mucosal immunity in mice after mucosal stimulation with cholera toxin. J. Immunol. 1984; 132 :2736-2741.
37. Elson CO. Cholera toxin and its subunits as potential oral adjuvants. Curr. Topics Microbiol. Immunol. 1989; 146 :29-33.
38. Lycke N, Holmgren J. Strong adjuvant properties of cholera toxin on gut mucosal immune responses to orally presented antigens. Immunology 1986; 59 :301-308.
39. Wilson AD, Stokes CR, Bourne FJ. Adjuvant effect of cholera toxin on the mucosal immune response to soluble proteins. Differences between mouse strains and protein antigens. Scand. J. Immunol. 1989; 29 :739-745.
40. Wilson AD, Clarke CJ, Stokes CR. Whole cholera toxin and B subunit act synergistically as an adjuvant for the mucosal immune response of mice to keyhole limpet haemocyanin. Scand. J. Immunol. 1990; 31 :443-451.
41. Lycke N, Tsuji T, Holmgren J. The adjuvant effect of *Vibrio cholerae and E. coli* heat labile enterotoxins is linked to the ability to stimulate cAMP. European Journal of Immunology 1992; 22 :2277-2281.
42. Lycke N, Karlsson U, Sjölander A, Magnusson K-E. The adjuvant action of cholera toxin is associated with an increased intestinal permeability for luminal antigens. Scandinavian Journal of Immunology 1991; 33 :691-698.
43. Gizurarson S, Tamura S, Kurata T, Hasiguchi K, Ogawa H. The effect of cholera toxin and cholera toxin B subunit on the nasal mucosa membrane. Vaccine 1991; 9 :825-832.
44. Bromander A, Holmgren J, Lycke N. Cholera toxin stimulates IL-1 production and enhances antigen presentation by macrophages in vitro. Journal of Immunology 1991; 146 :2908-2914.
45. Anastassiou ED, Yamada H, Francis ML, Mond JJ, Tsokos GC. Effects of cholera toxin on human B cells. Cholera toxin induces B cell surface DR expression while it inhibits anti-µ antibody-induced cell proliferation. J. Immunol. 1990; 145 :2375-2380.
46. Muñoz E, Zubiaga AM, Merrow M, Sauter NP, Huber BT. Cholera toxin discriminates between T helper 1 and 2 cells in T cell receptor-mediated activation: Role of cAMP in T cell proliferation. J. Exp. Med. 1990; 172 :95-103.
47. Lycke N, Strober W. Cholera toxin promotes B cell isotype differentiation. J. Immunol. 1989; 142 :3781-3787.
48. Wilson AD, Bailey M, Williams NA, Stokes CR. The *in vitro* production of cytokines by mucosal lymphocytes immunized by oral administration of keyhole limpet hemocyanin using cholera toxin as an adjuvant. European Journal of Immunology 1991; 21 :2333-2339.
49. Francis ML, Ryan J, Jobling MG, Holmes RK, Moss J, Mond JJ. Cyclic AMP-independent effects of cholera toxin on B cell activation. II. Binding of ganglioside G_{M1} induces B cell activation. Journal of Immunology 1992; 148 :1999-2005.
50. Woogen SD, Ealding W, Elson CO. Inhibition of murine lymphocyte proliferation by the B subunit of cholera toxin. Journal of Immunology 1987; 139 :3764-3770.
51. Garrone P, Banchereau J. Agonistic and antagonistic effects of cholera toxin on human B lymphocyte proliferation. Molecular Immunology 1993; 30 :627-635.
52. Haack BM, Emmrich F, Resch K. Cholera toxin inhibits T cell receptor signaling by covalent modification of the CD3-ζ subunit. Journal of Immunology 1993; 150 :2599-2606.
53. Abraham E, Robinson A. Oral immunization with bacterial polysaccharide and adjuvant enhances antigen-specific pulmonary secretory antibody response and resistance to pneumonia. Vaccine 1991; 9 :757-764.
54. Szu SC, Li X, Schneerson R, Vickers JH, Bryla D, Robbins JB. Comparative immunogenicities of Vi polysaccharide-protein conjugates composed of cholera toxin or its B subunit as a carrier bound to high- or lower-molecular-weight Vi. Infect. Immun. 1989; 57 :3823-3827.
55. Chen K-S, Strober W. Cholera holotoxin and its B subunit enhance Peyer's patch B cell responses induced by orally administered influenza virus: disproportionate cholera toxin enhancement of the IgA B cell response. Eur. J. Immunol. 1990; 20 :433-436.
56. Liang X, Lamm ME, Nedrud JG. Oral administration of cholera toxin-Sendai virus conjugate potentiates gut and respiratory immunity against Sendai virus. Journal of Immunology 1988; 141 :1495-1501.
57. Brimblecombe FSW, Cruicshank R, Masters PL, Reid DD, Stewart GT. Family studies of respiratory infections. British Medical Journal 1958; :119-128.
58. Masters PL, Brumfitt W, Mendez RL, Likar M. Bacterial flora of the upper respiratory tract in Paddington families, 1952-1954. Brit. Med. J. 1958; 1 :1200-1205.
59. Gwaltney JM, Sande MA, Austrian R, al. e. Spread of streptococcus pneumoniae in families: II Relation of transfer of *Streptococcus pneumoniae* to incidence of colds and serum antibody. J. Infect. Dis. 1975; 132 :62.
60. Russell MW, Prince SJ, Ligthart GJ, Mestecky J, Radl J. Comparison of salivary and serum antibodies to common environmental antigens in elderly, edentulous, and normal adult subjects. Aging Immunol. Infect. Dis. 1990; 2 :275-286.
61. Bessen D, Fischetti VA. Influence of intranasal immuniation with synthetic peptides corresponding to conserved epitopes of M protein on mucosal immunization by group A streptococci. Infect. Immun. 1988; 56 :2666-2672.
62. Hollingshead SK, Simecka JW, Michalek SM. Role of M protein in pharyngeal colonization by group A streptococci in rats. Infect. Immun. 1993; 61 :2277-2283.
63. Kauppi M, Eskola J, Kathty H. H. influenzae type b (Hib) conjugate vaccines induce mucosal IgA1 and IgA2 antibody responses in infants and children. ICAAC Abstracts 1993; 33 :174.
64. Briles DE, Forman C, Horowitz JC, Volanakis JE, Benjamin WH Jr., McDaniel LS, Eldridge J, Brooks J. Antipneumococcal effects of C-reactive protein and monoclonal antibodies to pneumococcal cell wall and capsular antigens. Infect. Immun. 1989; 57 :1457 - 1464.
65. Briles DE, Claflin JL, Schroer K, Forman C. Mouse IgG3 antibodies are highly protective against infection with *Streptococcus pneumoniae*. Nature 1981; 294 :88-90.
66. Lock RA, Paton JC, Hansman D. Comparative efficacy of pneumococcal neuraminidase and pneumolysin as immunogens protective against *Streptococcus pneumoniae*. Microb. Pathog. 1988; 5 :461-467.
67. Lock RA, Hansman D, Paton JC. Comparative efficacy of autolysin and pneumolysin as immunogens protecting mice against infection by *Streptococcus pneumoniae*. Microbial Pathogenesis 1992; 12 :137-143.
68. Converse GM III, Dillon HC Jr. Epidemiological studies of *Streptococcus pneumoniae* in infants: methods of isolating pneumococci. J. Clin. Micro. 1977; 5 :293-296.

## Claims

1. An intranasally administrable vaccine composition which confers protection against colonisation by S, pneumoniae in the nasopharynx of a host administered the composition, which comprises:
an effective amount of a pneumococcal surface protein A (PspA) in the form of a killed whole pneumococci, a pneumococcal lysate, an isolated and purified PspA or an immunogenic fragment thereof containing at least one protection-eliciting epitope,
an adjuvanting amount of the B subunit of cholera toxin, and
a pharmaceutical carrier therefor.

2. A composition comprising pneumococcal surface protein A (PspA) in the form of a killed whole pneumococci, a lysate of pneumococci or an isolated and purified PspA or an immunogenic fragment thereof, for preparation of an immunological mucosally administrable composition for use in a method which comprises administering to a host an immunising amount of said substance or composition in conjunction with an adjuvanting amount of the B subunit of cholera toxin (CTB).

3. A composition as claimed in Claim 2, wherein said composition is for use in a method of immunisation of a host against colonisation by S.pneumoniae in the nasopharynx by the administration of said composition intranasally.

4. Use of pneumococcal surface protein A (PspA) or at least one fragment of such PspA containing at least one protection eliciting epitope in the preparation of a medicament to immunise a host against pneumococcal infection by mucosal administration to the host of an immunising amount of said medicament.

5. Use as claimed in Claim 4, wherein said PspA is in the form of killed whole pneumococci or of a lysate of pneumococci.

6. Use as claimed in Claim 4, wherein said PspA is in the form of an isolated and purified PspA.

7. Use as claimed in any one of Claims 4 to 6, wherein said PspA is administered in conjunction with an adjuvant.

8. Use as claimed in Claim 7, wherein the adjuvant is the B subunit of cholera toxin.

9. Use as claimed in any one of Claims 4 to 8, wherein said mucosal administration is effected intranasally to provide protection to the host against both pneumococcal colonisation and systemic infection.

10. Use as claimed in any one of Claims 4 to 8, wherein said mucosal administration is effected intranasally to provide protection to the host against pulmonary infection.

11. Use as claimed in any one of Claims 4 to 8, wherein said mucosal adminstration is effected intranasally to provide protection to the host against infection starting as a pulmonary infection.

12. Use of pneumococcal surface protein A (PspA) in the form of a killed whole pneumococci, a lysate of pneumococci or an isolated and purified PspA or fragment thereof in the preparation of a medicament to immunise a host against colonisation with S. pneumoniae by intranasal administration to the host of an immunising amount of said medicament.

13. Use as claimed in Claim 12, wherein said PspA is administered intranasally in conjunction with an adjuvanting amount of the B subunit of cholera toxin (CTB).

14. Use as claimed in Claim 12 or Claim 13, to prevent carriage of pneumococci and prevent pneumococcal disease.

15. Use as claimed in Claim 12 or Claim 14, to prevent pulmonary infection and pneumococcal disease.

16. Use as claimed in Claim 12 or Claim 14, to prevent pulmonary infection and pneumococcal disease originating from pulmonary infection.

## Patentansprüche

1. Intranasal verabreichbare Impfstoffzusammensetzung, die Schutz gegen Kolonisierung durch S pneumoniae im Nasenrachenraum eines Wirts, dem die Zusammensetzung verabreicht wird, verleiht, umfassend:
eine wirksame Menge eines Pneumokokken-Oberflächenproteins A (PspA) in Form von abgetöteten ganzen Pneumokokken, eines Pneumokokken-Lysats, eines isolierten und gereinigten PspA oder eines immunogenen Fragments davon, das mindestens ein Schutz hervorrufendes Epitop enthält,
eine als Adjuvans wirkende Menge der B-Untereinheit von Choleratoxin, und
einen pharmazeutischen Träger dafür.

2. Zusammensetzung, umfassend Pneumokokken-Oberflächenprotein A (PspA) in Form von abgetöteten ganzen Pneumokokken, eines Lysats von Pneumokokken oder eines isolierten und gereinigten PspA oder eines immunogenen Fragments davon für die Herstellung einer immunologischen Zusammensetzung, die über die Schleimhaut verabreichbar ist, zur Verwendung in einem Verfahren, das die Verabreichung an einen Wirt einer immunisierenden Menge des Stoffes oder der Zusammensetzung in Verbindung mit einer als Adjuvans wirkenden Menge der B-Untereinheit von Choleratoxin (CTB) umfaßt.

3. Zusammensetzung nach Anspruch 2, wobei die Zusammensetzung zur Verwendung in einem Immunisierungsverfahren eines Wirtes gegen Kolonisierung durch S. pneumoniae im Nasenrachenraum durch die intranasale Verabreichung der Zusammensetzung dient.

4. Verwendung von Pneumokokken-Oberflächenprotein A (PspA) oder mindestens eines Fragments eines solchen PspA das mindestens ein Schutz hervorrufendes Epitop enthält, für die Herstellung eines Medikaments zur Immunisierung eines Wirtes gegen Pneumokokken-Infektion durch Verabreichung einer immunisierenden Menge des Medikaments über die Schleimhaut an den Wirt.

5. Verwendung nach Anspruch 4, wobei das PspA in Form von abgetöteten ganzen Pneumokokken oder als ein Lysat von Pneumokokken vorliegt.

6. Verwendung nach Anspruch 4, wobei das PspA in Form eines isolierten und gereinigten PspA vorliegt.

7. Verwendung nach einem der Ansprüche 4 bis 6, wobei das PspA in Verbindung mit einem Adjuvans verabreicht wird.

8. Verwendung nach Anspruch 7, wobei das Adjuvans die B-Untereinheit von Choleratoxin ist.

9. Verwendung nach einem der Ansprüche 4 bis 8, wobei die Verabreichung über die Schleimhaut intranasal erfolgt, um dem Wirt Schutz gegen sowohl Pneumokokken-Kolonisation als auch systemische Infektion zu verleihen.

10. Verwendung nach einem der Ansprüche 4 bis 8, wobei die Verabreichung über die Schleimhaut intranasal erfolgt, um dem Wirt Schutz gegen eine Lungeninfektion zu verleihen.

11. Verwendung nach einem der Ansprüche 4 bis 8, wobei die Verabreichung über die Schleimhaut intranasal erfolgt, um dem Wirt Schutz gegen eine Infektion zu verleihen, die als Lungeninfektion beginnt.

12. Verwendung eines Pneumokokken-Oberflächenproteins A (PspA) in Form von abgetöteten ganzen Pneumokokken oder eines Lysats von Pneumokokken oder eines isolierten und gereinigten PspA oder eines Fragmentes davon für die Herstellung eines Medikaments zur Immunisierung eines Wirts gegen Kolonisierung mit S. pneumoniae durch intranasale Verabreichung einer immunisierenden Menge des Medikaments an den Wirt.

13. Verwendung nach Anspruch 12, wobei das PspA intranasal in Verbindung mit einer als Adjuvans wirkenden Menge der B-Untereinheit von Choleratoxin (CTB) verabreicht wird.

14. Verwendung nach Anspruch 12 oder 13, zur Vorbeugung gegen Pneumokokken-Träger und zur Vorbeugung einer Pneumokokken-Erkrankung.

15. Verwendung nach Anspruch 12 oder 14, zur Vorbeugung gegen eine Lungeninfektion und eine Pneumokokken-Erkrankung.

16. Verwendung nach Anspruch 12 oder 14, zur Vorbeugung gegen eine Lungeninfektion und eine Pneumokokken-Erkrankung, die aus einer Lungeninfektion hervorgeht.

## Revendications

1. Composition de vaccin à administrer par voie intranasale qui confère une protection contre la colonisation par S. pneumoniae dans le rhino-pharynx d'un hôte à qui l'on a administré la composition, qui comprend:
une quantité efficace d'une protéine de surface pneumococcique A (PspA) sous forme de pneumocoques entiers tués, d'un lysat pneumococcique, d'une PspA isolée et purifiée ou d'un de ses fragments immunogènes contenant au moins un épitope déclenchant la protection,
une quantité adjuvante de la sous-unité B de la toxine cholérique, et un véhicule pharmaceutique pour ces composés.

2. Composition comprenant une protéine de surface pneumococcique A (PspA) sous forme de pneumocoques entiers tués, d'un lysat de pneumocoques ou d'une PspA isolée et purifiée ou un de ses fragments immunogènes, pour la préparation d'une composition immunologique à administrer par les muqueuses et à utiliser dans un procédé qui comprend l'administration à un hôte d'une quantité immunisante de ladite substance ou composition en association avec une quantité adjuvante de la sous-unité B de la toxine cholérique (CTB).

3. Composition selon la revendication 2, dans laquelle ladite composition est à utiliser dans un procédé d'immunisation d'un hôte contre la colonisation par S. pneumoniae dans le rhino-phaynx par administration de ladite composition par voie intranasale.

4. Utilisation de la protéine de surface pneumococcique A (PspA) ou au moins d'un fragment de ladite PspA contenant au moins un épitope déclenchant la protection dans la préparation d'un médicament pour immuniser un hôte contre une infection à pneumocoques, par administration par les muqueuses à l'hôte d'une quantité immunisante dudit médicament.

5. Utilisation selon la revendication 4, dans laquelle ladite PspA est sous forme de pneumocoques entiers tués ou d'un lysat de pneumocoques.

6. Utilisation selon la revendication 4, dans laquelle ladite PspA est sous forme d'une PspA isolée et purifiée.

7. Utilisation selon l'une quelconque des revendications 4 à 6, dans laquelle ladite PspA est administrée en association avec un adjuvant.

8. Utilisation selon la revendication 7, dans laquelle l'adjuvant est la sous-unité B de la toxine cholérique.

9. Utilisation selon l'une quelconque des revendications 4 à 8, dans laquelle ladite administration par les muqueuses est effectuée par voie intranasale pour procurer une protection de l'hôte à la fois contre une colonisation par des pneumocoques et contre une infection systémique à pneumocoques.

10. Utilisation selon l'une quelconque des revendications 4 à 8, dans laquelle ladite administration par les muqueuses est effectuée par voie intranasale pour procurer une protection de l'hôte contre une infection pulmonaire.

11. Utilisation selon l'une quelconque des revendications 4 à 8, dans laquelle ladite administration par les muqueuses est effectuée par voie intranasale pour procurer une protection de l'hôte contre une infection commençant par une infection pulmonaire.

12. Utilisation de la protéine de surface pneumococcique A (PspA) sous forme de pneumocoques entiers tués, d'un lysat de pneumocoques ou d'une PspA isolée et purifiée ou d'un de ses fragments dans la préparation d'un médicament pour immuniser un hôte contre une colonisation par S. pneumoniae par administration intranasale à l'hôte d'une quantité immunisante dudit médicament.

13. Utilisation selon la revendication 12, dans laquelle ladite PspA est administrée par voie intranasale en association avec une quantité adjuvante de la sous-unité B de la toxine cholérique (CTB).

14. Utilisation selon la revendication 12 ou la revendication 13, pour empêcher le transport des pneumocoques et empêcher les maladies à pneumocoques.

15. Utilisation selon la revendication 12 ou la revendication 14, pour empêcher une infection pulmonaire et une maladie à pneumocoques.

16. Utilisation selon la revendication 12 ou la revendication 14, pour empêcher une infection pulmonaire et une maladie à pneumocoques provenant d'une infection pulmonaire.
